Europäisches Patentamt

**European Patent Office**

**Office européen des brevets**

(11) Veröffentlichungsnummer: **0 440 022 A2**

# EUROPÄISCHE PATENTANMELDUNG

(21) Anmeldenummer: **91100199.8**

(51) Int. Cl.5: **A24B 15/24**

(22) Anmeldetag: **08.01.91**

(30) Priorität: **31.01.90 DE 4002784**

(43) Veröffentlichungstag der Anmeldung:
**07.08.91 Patentblatt 91/32**

(84) Benannte Vertragsstaaten:
**AT BE CH DE DK ES FR GB GR IT LI LU NL SE**

(71) Anmelder: **B.A.T. Cigaretten-Fabriken GmbH**
**Alsterufer 4**
**W-2000 Hamburg 36(DE)**

(72) Erfinder: **Heemann, Volker, Dipl.-Chem. Dr.**
**Klosterbergenstrasse 42**
**W-2057 Reinbek(DE)**
Erfinder: **Schmekel, Gerald, Dipl.-Ing.**
**Schumacherstrasse 72**
**W-2200 Elmshorn(DE)**
Erfinder: **Ehling, Uwe, Dipl.-Ing.**
**Schumacherstrasse 78**
**W-2200 Elmshorn(DE)**
Erfinder: **Hauser, Bernhard, Dr. Dipl.-Chem.**
**Distelkoppel 12**
**W-2000 Schenefeld(DE)**
Erfinder: **Koene, Casper Henk**
**Stadtbahnstrasse 78a**
**W-2000 Hamburg 65(DE)**
Erfinder: **Rabitz, Helge**
**Noldering 17**
**W-2000 Hamburg 60(DE)**

(74) Vertreter: **Schwabe - Sandmair - Marx**
**Stuntzstrasse 16**
**W-8000 München 80(DE)**

(54) **Verfahren zur Extraktion von Tabakalkaloiden.**

(57) Beschrieben wird ein Verfahren zur Extraktion von Tabakalkaloiden aus Tabak, bei dem man die Tabakalkaloide mit Kohlendioxid unter überkritischen Bedingungen aus dem Tabak extrahiert und die Tabakalkaloide aus dem Kohlendioxid abtrennt, wobei man in dem Kohlendioxid für die Extraktion der Tabakalkaloide aus dem Tabak eine feste 2- bis 3-wertige organische Säure mit insgesamt 2 bis 6 Kohlenstoffatomen und/ oder deren Monoalkali- oder Monoammonium-Salze löst, um die Extraktionszeiten relevant zu verkürzen.

## VERFAHREN ZUR EXTRAKTION VON TABAKALKALOIDEN

Die Erfindung betrifft ein Verfahren zur Extraktion von Tabakalkaloiden aus Tabak. Insbesondere betrifft die Erfindung ein Verfahren, mit dem man Nikotin aus Tabak extrahiert.

Die Extraktion von Tabakalkaloiden aus Tabak gehört bereits seit langem zum Stand der Technik. Extraktive Verfahren unter Verwendung organischer oder wässriger Lösungsmittel spielen dabei eine wichtige Rolle. Insbesondere die Extraktion von Nikotin unter Verwendung derartiger organischer oder wässriger Lösungsmittel ist Ziel verschiedener Prozesse. Der Nachteil dieser Verfahren ist, daß die Extraktion unspezifisch verläuft und mehr oder weniger große Mengen von Aromastoffen zusammen mit dem Nikotin aus dem Tabak extrahiert werden.

Darüber hinaus wirken sich solche Extraktionsverfahren auch negativ auf die Struktur des Tabaks aus und beeinflussen in unerwünschter Weise den Geschmack des Rauches, der beim Abbrand derartigen Tabaks entsteht. Die Auftrennung der bei der Extraktion gewonnenen Extrakte und die anschließende Rekombination der Aromastoffe enthaltenden Fraktion mit dem Tabak ist sehr aufwendig.

Selektiver lassen sich Tabakalkaloide, insbesondere Nikotin, mit Lösungsmitteln im überkritischen Zustand extrahieren. Hierbei wurde vorzugsweise Kohlendioxid eingesetzt. Die DE-PS 2 043 537 beschreibt ein Verfahren, bei dem Nikotin mittels $CO_2$ im überkritischen Zustand aus Tabak extrahiert wird. Die Abscheidung des Nikotins kann entweder an Sorbenzien erfolgen oder durch Entspannung der Extrakt-Mischung erreicht werden. Aufgrund der relativ schlechten Löslichkeit von Nikotin sind lange Extraktionszeiten erforderlich. Diese wirken sich nachteilig auf den Rauchgeschmack des späteren Tabak-Abbrandes aus.

In der DE-PS 2 142 205 wird ein mehrstufiges Verfahren zur Entfernung von Nikotin aus Tabak beschrieben. Darbei werden zunächst die Aromastoffe aus trockenem Tabak mit Kohlendioxid im überkritischen Zustand extrahiert. Danach wird der Tabak mit feuchtem Kohlendioxid im überkritischen Zustand in Kontakt gebracht, wobei Nikotin entzogen wird. Im dritten Verfahrensschritt wird der Tabak wieder mit den Aromastoffen versetzt, die im ersten Schritt extrahiert worden waren. Die langen Prozeßzeiten führen - wie auch im oben beschriebenen Verfahren - zu Geschmackseinbußen und verhindern eine wirtschaftliche Verfahrensführung.

Zur Verringerung der Koextraktion von Aromastoffen bei der Entnikotinisierung kann ein Gemisch aus Stickstoff und Kohlendioxid unter überkritischen Bedingungen zur Extraktion eingesetzt werden (DE-OS 33 34 736). In der DE-OS 2 844 781 wird vorgeschlagen, durch eine Kombination einer gasförmigen (Kohlendioxid) und einer flüssigen (Ethanol) Komponente die Effektivität und Spezifität des Extraktionsschrittes zu erhöhen. Auch diese Verfahren haben den Nachteil, daß aufgrund der geringen Löslichkeit von Nikotin in überkritischem Kohlendioxid Prozeßzeiten von mehreren Stunden erforderlich sind, die einer wirtschaftlichen Anwendung dieses Extraktionsverfahrens entgegenstehen. Die Extraktion auf den angegebenen Wegen führt darüber hinaus zu erheblichen Einbußen in Tabakqualität und Rauchgeschmack.

Die EP-A 0 280 817 beschreibt ein Verfahren zur Extraktion von Nikotin aus Tabak und Tabak-Mischungen unter Verwendung von überkritischen oder flüssigen Extraktionsmitteln. Dabei wird der Tabak mit Extraktionsmitteln, u.a. auch mit Kohlendioxid, in Kontakt gebracht, um Nikotin und auch andere Tabak-Inhaltsstoffe zu extrahieren. Das Nikotin wird aus dem Extrakt dadurch abgetrennt, daß man den Extrakt ein Gefäß durchströmen läßt, das eine nicht flüchtige Säure bzw. ein Salz dieser Säure enthält. Die Aroma-Komponenten passieren dieses Gefäß zusammen mit dem Extraktionsmittel, während das Nikotin in dem Gefäß an der Säure bzw. dem Salz chemisch gebunden und dadurch von dem Kohlendioxid abgetrennt wird.

Ein Nachteil des in der EP-A 0 280 817 offenbarten Verfahrens liegt darin, daß die zur Bindung des Nikotins erforderliche nichtflüchtige Säure bzw. ein Salz dieser Säure auf geeignete Trägermaterialien, wie beispielsweise Schalen von Kakaobohnen, aufgezogen werden muß. Darüber hinaus müssen die Behandlungsgefäße, insbesondere die Druckbehälter sehr groß dimensioniert werden, da zur Abtrennung des Nikotins aus dem Extrakt die Säure bzw. ihr Salz (und damit auch die erforderlichen Trägermaterialien) im Verhältnis von mindestens 1:1, vorzugsweise jedoch von > 1:1, eingesetzt werden müssen.

In der EP-A 0 280 817 wird darüber hinaus festgestellt, daß die zur Abtrennung des Nikotins aus dem Extrakt durch chemische Bindung geeigneten nichtflüchtigen Säuren in dem verwendeten Extraktionslösemittel unter den Extraktions-Bedingungen nicht löslich sein dürfen. Besonders bevorzugt werden daher solche Salze nichtflüchtiger Säuren, die eine besonders geringe Löslichkeit haben. Dadurch soll offenbar vermieden werden, daß Säure bzw. Salz den Trennbehälter verlassen und in den Extraktions-Kreislauf eindringen kann.

Der vorliegenden Erfindung liegt die Aufgabe zugrunde, ein Verfahren bereitzustellen, mit dem Tabakalkaloide aus Tabak in einfacher und das Tabak-Rohmaterial schonender Weise durch Extraktion abgetrennt werden können. Insbesondere soll ein Verfahren vorgeschlagen werden, das dem Tabak Aromastoffe nicht entzieht, sondern eine selektive Extraktion bestimmter Tabakalkaloide, insbesondere des Nikotins, bewirken kann. Unter dem Gesichtspunkt der späteren Verwendung des Tabaks waren aufgabengemäß insbesondere kurze Behandlungszeiten, die eine Verschlechterung der Tabakqualität nicht befürchten lassen, und die Verwendung physiologisch unbedenklicher Lösungsmittel mit niedrigem Kilogramm-Preis zu berücksichtigen.

Überraschenderweise wurde gefunden, daß bestimmte nichtflüchtige organische Säuren bzw. deren Monoalkalimetall-Salze eine bisher nicht bekannte Löslichkeit in überkritischem Kohlendioxid (Druck: etwa 250 bar; Temperatur: etwa 70 °C) besitzen. Ein weiteres überraschendes Ergebnis der Erfindung besteht darin, daß sich unter den Prozeßbedingungen die Löslichkeit von Nikotin in überkritischem Kohlendioxid um den Faktor 3 bis 5 erhöht, wenn das Kohlendioxid gewisse Mengen derartiger nichtflüchtiger organischer Säuren bzw. deren Monoalkalimetall- oder Monoammonium-Salze enthält. Dadurch lassen sich die zur Extraktion von Tabakalkaloiden, insbesondere Nikotin, erforderlichen Extraktionszeiten verringern, so daß die verfahrenstechnisch-ökonomischen und geschmacklichen Nachteile der bekannten Verfahren überwunden werden.

Die Erfindung betrifft ein Verfahren zur Extraktion von Tabakalkaloiden aus Tabak, bei dem man die Tabakalkaloide mit Kohlendioxid unter überkritischen Bedingungen aus dem Tabak extrahiert und die Tabakalkaloide aus dem Kohlendioxid abtrennt, wobei ein solches Verfahren dadurch gekennzeichnet ist, daß man in dem Kohlendioxid für die Extraktion der Tabakalkaloide aus dem Tabak eine feste, 2- bis 3-wertige organische Säure mit insgesamt 2 bis 6 Kohlenstoffatomen und/ oder deren Monoalkali- oder Monoammonium-Salze löst.

Mit dem erfindungsgemäßen Verfahren können aus Tabak beliebiger Provenienz unterschiedliche Tabakalkaloide selektiv extrahiert werden. Besonders bevorzugt wird das Nikotin aus Tabak extrahiert. Wenn auch das erfindungsgemäße Verfahren nicht auf die Extraktion von Nikotin beschränkt ist, wird es doch nachfolgend an diesem bevorzugten Beispiel näher erläutert.

Erfindungsgemäß wird in dem Kohlendioxid für die Extraktion der Tabakalkaloide aus dem Tabak eine feste, 2- bis 3-wertige organische Säure mit insgesamt 2 bis 6 Kohlenstoffatomen gelöst. Beispiele solcher Säuren sind Oxalsäure, Malonsäure, Bernsteinsäure, Glutarsäure, Adipinsäure, Äpfelsäure, Weinsäure, Schleimsäure und Citronensäure.

Es entspricht einer bevorzugten Ausführungsform des erfindungsgemäßen Verfahrens, anstelle der genannten organischen Säuren oder auch zusammen mit diesen Säuren ein oder mehrere Monoalkali- oder Monoammonium-Salze dieser Säuren in dem Kohlendioxid für die Extraktion der Tabakalkaloide aus dem Tabak zu lösen. Als Monoalkali-Salze sind Lithium-, Natrium-, Kalium- oder Rubidium-Salze verwendbar. Aufgrund der leichten Verfügbarkeit werden in erster Linie die Natriumund/oder Kalium-Salze verwendet. In gleicher Weise sind jedoch auch Monoammonium-Salze einsetzbar.

In einer besonders bevorzugten Ausführungsform des Verfahrens löst man in dem Kohlendioxid für die Extraktion der Tabakalkaloide aus dem Tabak eine organische Säure aus der Gruppe Oxalsäure, Malonsäure, Äpfelsäure, Weinsäure und Citronensäure und/oder deren Monoalkali- oder MonoammoniumSalze. Besonders gute Ergebnisse lassen sich bei Zusatz der Monoalkalimetall-Oxalate, -Malonate, -Malate, -Tartrate oder -Citrate erzielen. Die Löslichkeit von Tabakalkaloiden, insbesondere von Nikotin, wird überraschenderweise insbesondere dann signifikant erhöht, wenn man dem für die Extraktion der Tabakalkaloide aus dem Tabak vorgesehenen Kohlendioxid Kaliumcitrat zusetzt.

Die Menge an dem Kohlendioxid zugesetzter organischer Säure bzw. an Salz der entsprechenden Säure ist insofern kritisch, als eine Säuremenge bzw. eine Salzmenge zugesetzt werden muß, die zu einer für die praktische Anwendung genügend hohen Verbesserung der Löslichkeit der Tabakalkaloide, insbesondere des Nikotins, im Extraktionsmittel Koh- lendioxid führt. Es wird erfindungsgemäß bevorzugt, dem Kohlendioxid für die Extraktion der Tabakalkaloide aus dem Tabak die organische Säure oder ihre Salze in Sättigungsmengen zuzusetzen, d.h. in solchen Mengen, die zu einer Sättigung der entsprechenden Verbindung bzw. Verbindungen im Kohlendioxid unter Prozeßbedingungen führen. Bei Sättigung wird die größtmögliche Erhöhung der Löslichkeit von beispielsweise Nikotin im überkritischen Kohlendioxid erreicht, so daß die besten Extraktionsergebnisse erzielt werden können. Dies ist beispielsweise für Kaliummonocitrat etwa 0,02 % Gew.-% bezogen auf das eingesetzte $CO_2$.

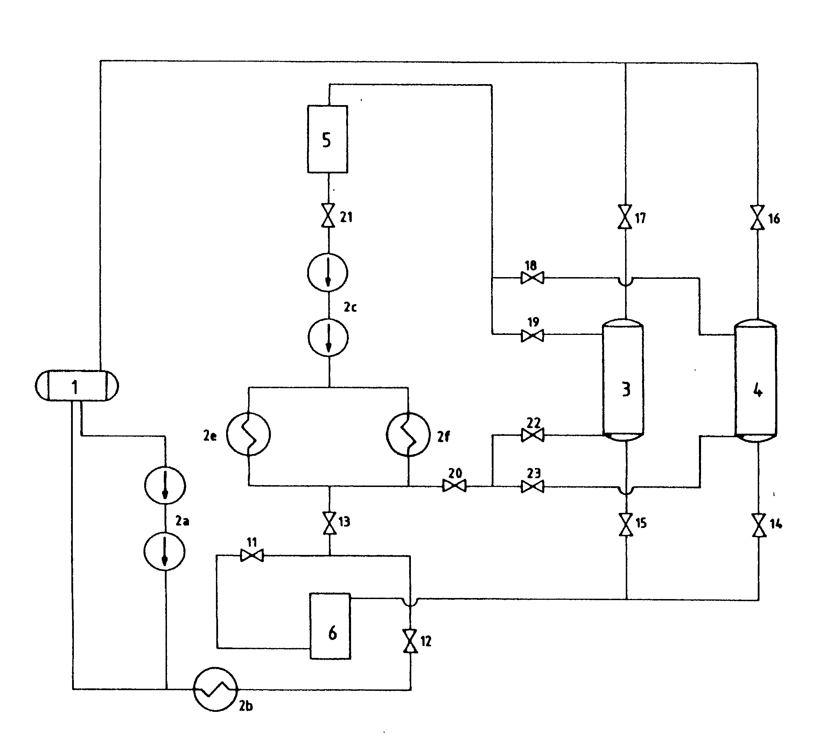

Die Erfindung wird nachfolgend unter Bezugnahme auf die beiliegende einzige Figur näher erläutert, die ein Fließbild des erfindungsgemäßen Verfahrens zeigt.

Dieses Verfahren wird in an sich bekannter Weise in einer Anlage durchgeführt, die den Betrieb unter Bedingungen gestattet, bei denen das Extraktions-Lösungsmittel Kohlendioxid im überkritischen Zustand vorliegt. Druckfeste Behälter und Anlagenteile entsprechender Auslegung sind dem Fachmann aus dem Stand der Technik bekannt.

In bevorzugter Führung des erfindungsgemäßen Verfahrens wird das flüssige Kohlendioxid aus einem Arbeitstank 1 über eine oder mehrere Flüssigkeitspumpen 2a in die Anlage eingespeist. Dabei wird das Kohlendioxid über einen Wärmetauscher 2b geleitet, um es auf Prozeßtemperatur zu bringen. Diese liegt in der Regel bei etwa 70° C. Es sind jedoch auch etwas höhere oder niedrigere Temperaturen denkbar.

Vor dem Kontakt mit dem Tabak wird das Kohlendioxid mit einer festen, 2- bis 3-wertigen organischen Säure mit insgesamt 2 bis 6 Kohlenstoffatomen und/oder mit einem oder mehreren Monoalkali- oder Monoammonium-Salzen einer derartigen organischen Säure versetzt. Bei der in der Figur dargestellten Weise geschieht dies dadurch, daß man aus dem Grundzustand (alle Ventile geschlossen) durch öffnen der Ventile 12 und 11 das überkritische Kohlendioxid in einen Säure-bzw. Salzbehälter 6 strömen läßt. Der Säure- bzw. Salzbehälter 6 kann dabei in axialer Richtung oder auch in radialer Richtung von dem Kohlendioxid angeströmt werden. Der Behälter 6 ist an den Einlaß- bzw. Auslaßseiten mit einer für diesen Zweck geeigneten Siebplatte versehen, die ein Ausströmen der festen Säure bzw. des festen Salzes verhindert, jedoch einen freien Durchfluß des überkritischen Kohlendioxids, gegebenenfalls des überkritischen, mit Säure bzw. Salz beladenen Kohlendioxids ermöglicht. Gegebenenfalls kann das Kohlendioxid den Behälter mehrmals durchströmen.

Das mit der Säure und/oder einem Salz dieser Säure versetzte Kohlendioxid wird nach öffnen der Ventile 14, 15 mittels der Flüssigkeitspumpen 2a aus dem Behälter 6 zu den Tabakbehältern 3 bzw. 4 gepumpt.

Das mit der Säure bzw. einem oder mehreren ihrer Salze beladene Kohlendioxid wird nachfolgend zur Extraktion des Tabakalkaloids, bevorzugt des Nikotins, mit dem Tabak in innigen Kontakt gebracht. Dies geschieht in der Weise, daß man es durch einen oder gegebenenfalls auch mehrere, in diesem Fall parallel geschaltete Tabak-Extraktionsbehälter 3, 4 führt. Diese enthalten den an sich aus dem Stand der Technik bekannten, vorkonditionierten Tabak oder Rohtabak. Soll nur ein Behälter durchströmt werden, beispielsweise der Behälter 3, müssen die Ventile 14, 16, 17 und 18 geschlossen und die Ventile 15 und 19 geöffnet sein. Bei gleichzeitigem Einströmen in beide Behälter sind alle Ventile 14, 15, 18 und 19 geöffnet.

Die Anströmung der Tabak-Extraktionsbehälter 3 und 4 kann in beliebiger, aus dem Stand der Technik bekannter Weise erfolgen. Denkbar sind hier beispielsweise eine axiale oder auch eine radiale Anströmung. Entscheidend ist, daß der Tabak in innigen Kontakt mit der Mischung aus überkritischem Kohlendioxid und zugesetzter, darin gelöster Säure bzw. zugesetztem darin gelöstem Monoalkali- bzw. Monoammonium-Salz gelangt. Die Kontaktzeit, d.h. diejenige Zeit, während der eine Extraktion des bzw. der Tabakalkaloide aus dem Tabak erfolgt, ist wesentlich kürzer als bei herkömmlichen Verfahren. Sie liegt erfindungsgemäß im Bereich von etwa 30 bis 60 Minuten. Die Kontaktzeit ist jedoch bei einer Variation der anderen Prozeßparameter variabel und kann geringfügig mehr als 60 Minuten betragen. Es sind jedoch auch deutlich kürzere Kontaktzeiten denkbar.

Die Extraktmischung aus Kohlendioxid, darin gelöster Säure bzw. darin gelöstem Salz und Tabakalkaloid, insbesondere Nikotin, wird nachfolgend einer Vorrichtung 5 zur Abtrennung der Tabakalkaloide aus der Mischung zugeführt. Als Vorrichtung zur Abtrennung der Tabakalkaloide aus der Extraktmischung wählt man mit Vorteil Gefäße, in denen man die Tabakalkaloide mittels eines Adsorbens abtrennen kann. Als Adsorbenzien kommen bevorzugt Aktivkohle oder zur Adsorption geeignete Aluminiumsilikate infrage. Von letzteren haben sich insbesondere Zeolite bewährt. Mit Zeoliten befüllte Adsorber gestatten eine nahezu vollständige Abtrennung von Tabakalkaloiden, insbesondere von Nikotin, in dem Austauschbehälter 5. Das mit der organischen Säure oder ihrem Salz beladene $CO_2$ wird an solchen Zeolit-Austauscher-Materialien nicht adsorbiert, sondern unverändert dem Kreislauf wieder zugeführt.

Es ist jedoch auch möglich, den Austauschbehälter 5 mit einem Chemisorbens zu befüllen und die Tabakalkaloide mittels eines Chemisorbens aus der Extrakt-Mischung abzutrennen. Bewährte und damit bevorzugte Chemisorbenzien sind Ionenaustauscher, besonders saure Ionenaustauscher. Die Passage der Extraktmischung durch einen mit einem sauren Ionenaustauscher befüllten Austauschbehälter 5 führt ebenfalls zu einer nahezu vollständigen Abtrennung der Tabakalkaloide, insbesondere des Nikotins, aus der Extraktmischung. Den Behälter 5 verlassen damit nur das überkritische Kohlendioxid und die darin gelöste Säure bzw. ihr Salz.

Gemäß einer bevorzugten Ausführungsform des Verfahrens wird die Kohlendioxid-Säure- bzw. -Salz-Mischung bei Verschluß der Ventile 13, 14, 15 und öffnung der Ventile 20, 22, 23 (nach dem Abschalten der Pumpen 2a und dem Schließen des Ventils 12) mittels der Pumpen 2c unmittelbar auf den bzw. die Tabakbehälter geleitet. Falls erforderlich, kann natürlich unter Hinzuschalten des Behälters 6 (Ventile 20, 22, 23 geschlossen, Ventile 13, 11, 14 und/oder 15 geöffnet) eine Nachsättigung erfolgen.

Die Nachsättigung ist normalerweise nicht erforderlich, da Salz weder vom Tabak noch vom Adsorbens bzw. Chemisorbens aufgenommen wird und sich die gute Löslichkeit der Kohlendioxid/Säurebzw. -Salz/Mischung für das Tabakalkaloid, insbesondere das Nikotin, im wesentlichen nicht ändert. Hierdurch wird die Kohlendioxid/Säure- bzw. -Salz/ Mischung im Kreislauf geführt, und der Extraktionszyklus beginnt von neuem.

Um die Anlage in den Ausgangszustand zu bringen, ist es notwendig, den Druckausgleich zwischen Arbeitstank 1 und dem oder den Behälter(n) 3, 4 durch Abkühlung und über ein nicht dargestelltes Ventil herzustellen. Die Ventile 16, 17 dienen der Verdrängung von $CO_2$-Flüssigkeit durch $CO_2$-Gas: Mit Hilfe der Pumpen 2c wird nämlich über eine nicht dargestellte Rohrleitung das flüssige $CO_2$ aus den Behältern 3, 4 in den Arbeitstank 1 zurückgepumpt. Das dann in den Behältern verbleibende $CO_2$-Gas wird über ein nicht dargestelltes Entspannungsventil abgelassen.

Die in der Figur dargestellte, bevorzugte Führung des erfindungsgemäßen Verfahrens mit zwei Tabak-Extraktionsbehältern ermöglicht einen quasi-kontinuierlichen Betrieb. Die Zahl der Tabak-Extraktionsbehälter ist verständlicherweise nicht auf zwei begrenzt. Wie bereits oben angedeutet, ist jedoch auch ein diskontinuierlicher Betrieb möglich; in diesem Fall reicht ein einziger Tabakextraktionsbehälter 3 aus.

Der Vorteil des erfindungsgemäßen Verfahrens gegenüber aus dem Stand der Technik bekannten Verfahren ist darin zu sehen, daß infolge der Verbesserung der Löslichkeit der Tabakalkaloide, insbesondere des Nikotins, im Extraktions-Lösungsmittel Kohlendioxid die Extraktionszeit drastisch verkürzt werden kann. Die Säure bzw. ihr Salz erhöhen die Löslichkeit von Nikotin in überkritischem Kohlendioxid um den Faktor 3 bis 5 (je nach Prozeßbedingungen). Die Extraktionszeiten sinken damit auf etwa 30 Minuten, wodurch gleichzeitig jede Verschlechterung der Qualität des behandelten Tabaks vermieden wird.

Im Gegensatz zum Stand der Technik wird die Säure bzw. ihr Salz nicht als Adsorptionsmittel zur Abscheidung des Nikotins aus der Extrakt-Mischung benutzt. Vielmehr erhöht sie die Löslichkeit der Tabakalkaloide, insbesondere des Nikotins, in dem Kohlendioxid. Die Säure bzw. das Salz werden damit auch nicht verbraucht bzw. aus dem Verfahren abgetrennt. Vielmehr wird das in der Extrakt-Mischung gelöste Tabakalkaloid bzw. Nikotin im Austauschbehälter abgeschieden, ohne daß es zu einer nennenswerten Abscheidung der Säure bzw. ihres Salzes im Austauschbehälter 5 kommt. Die organische Säure bzw. das Salz werden also zusammen mit dem Kohlendioxid im Kreislauf geführt.

Die Erfindung wird nachfolgend durch die Beispiele weiter erläutert.

## Beispiel 1

Es wurden 25 kg Schnittabak mit einer Feuchte von 25 % und einem Ausgangs-Nikotingehalt von 1,98 % eingesetzt. Überkritisches $CO_2$, das mit 1,5 kg Kaliummonocitrat im Vorratsbehälter versetzt und so gehalten wurde, daß immer eine Sättigung des Salzes im Kohlendioxid festzustellen war, wurde mit einem Volumenstrom von 5,2 m³/h in einer Anlage gemäß der Figur geführt. Die Extraktionstemperatur lag bei 70°C; der Druck betrug 250 bar. Der Massenstrom $CO_2$, bezogen auf die trokkene Tabakmasse, lag bei 208 h$^{-1}$. Der Nikotin-Gehalt im $CO_2$ lag nach Einstellung der Extraktionsbedingungen bei 70 mg/kg. Die Extrakt-Mischung wurde dem Austauschbehälter zugeführt, der mit 9 kg eines sauren Ionenaustauschers als Chemisorbens gefüllt war. Das Nikotin wurde dem Kohlendioxid vollständig entzogen. Nach einer Extraktionszeit von 30 Minuten betrug der Endnikotingehalt des Tabaks etwa 0,20 %.

Die Restmenge Kaliummonocitrat im Vorratsbehälter nach Versuchsende betrug noch 1,1 kg.

Während einer Extraktionszeit von nur 30 Minuten konnte also dem eingesetzten Tabak nahezu 90 % des Nikotins entzogen und dieses Nikotin aus der Extrakt-Mischung vollständig entfernt werden.

## Beispiel 2

Es wurde wie in Beispiel 1 verfahren. Jedoch wurden 100 kg Tabak mit einem Nikotingehalt von 2,50 % eingesetzt.

Die Extrakt-Mischung wurde danach an 10 kg Ionenaustauscher vollständig entnikotinisiert.

## Vergleichsbeispiel

Es wurde wie in Beispiel 1 verfahren. Jedoch war dem Extraktionsmittel Kohlendioxid keine Säure bzw. kein Salz einer solchen Säure zugesetzt. Der Nikotin-Gehalt in $CO_2$ lag nach Einstellung der Extraktionsbedingungen bei 17 mg/kg. Die Extraktionszeit bis zum Erreichen eines End-Nikotingehaltes von 0,18 % lag bei 14 Stunden.

Bei dem Verfahren nach dem Stand der Technik wurde somit dasselbe Ergebnis (End-Nikotingehalt im Tabak: etwa 0,2 %) unter gleichen Verfahrensbedingungen in einer Zeit erreicht, die 28-mal länger ist als die Extraktionszeit beim erfindungsgemäßen Vorgehen.

## Patentansprüche

1. Verfahren zur Extraktion von Tabakalkaloiden aus Tabak, bei dem man
   a) die Tabakalkaloide mit Kohlendioxid unter überkritischen Bedingungen aus dem Tabak extrahiert und

b) die Tabakalkaloide aus dem Kohlendioxid abtrennt,

dadurch **gekennzeichnet,** daß man in dem Kohlendioxid für die Extraktion der Tabakalkaloide aus dem Tabak eine feste, 2- bis 3- wertige organische Säure mit insgesamt 2 bis 6 Kohlenstoffatomen und/oder deren Monoalkali- oder Monoammoniumsalze löst.

2. Verfahren nach Anspruch 1, dadurch **gekennzeichnet,** daß Nikotin aus Tabak extrahiert wird.

3. Verfahren nach einem der Ansprüche 1 oder 2, dadurch **gekennzeichnet,** daß die Tabakalkaloide mittels eines Adsorbens von dem Kohlendioxid abgetrennt werden.

4. Verfahren nach Anspruch 3, dadurch **gekennzeichnet,** daß als Adsorbens Aktivkohle oder Aluminiumsilikate, bevorzugt Zeolite, verwendet werden.

5. Verfahren nach einem der Ansprüche 1 bis 3, dadurch **gekennzeichnet,** daß die Tabakalkaloide mittels eines Chemisorbens von dem Kohlendioxid abgetrennt werden.

6. Verfahren nach Anspruch 5, dadurch **gekennzeichnet,** daß das Chemisorbens ein Ionenaustauscher, bevorzugt ein saurer Ionenaustauscher, ist.

7. Verfahren nach einem oder mehreren der Ansprüche 1 bis 6, dadurch **gekennzeichnet,** daß man in dem Kohlendioxid für die Extraktion der Tabakalkaloide aus dem Tabak eine organische Säure aus der Gruppe Oxalsäure, Malonsäure, Bernsteinsäure, Glutarsäure, Adipinsäure, Äpfelsäure, Weinsäure, Schleimsäure und Citronensäure und/oder deren Monoalkali - oder Monoammoniumsalze löst.

8. Verfahren nach Anspruch 7, dadurch **gekennzeichnet,** daß man dem Kohlendioxid eine Verbindung aus der Gruppe Monoalkalimetall-Oxalate, -Malonate, -Malate, -Tartrate und - Citrate, bevorzugt Monoalkalimetall-Citrate, zusetzt.

9. Verfahren nach Anspruch 7 oder Anspruch 8, dadurch **gekennzeichnet,** daß man dem Kohlendioxid Kaliummonocitrat zusetzt.

10. Verfahren nach einem oder mehreren der Ansprüche 1 bis 9, dadurch **gekennzeichnet,** daß man dem Kohlendioxid für die Extraktion der Tabakalkaloide aus dem Tabak die organische Säure oder ihre Salze in Sättigungsmengen zusetzt.

11. Verfahren nach Anspruch 10, dadurch **gekennzeichnet,** daß die Konzentration von Kaliummonocitrat im Kohlendioxid etwa 0,02 Gew.-% beträgt.

12. Verfahren nach einem oder mehreren der Ansprüche 1 bis 11, dadurch **gekennzeichnet,** daß man das Kohlendioxid vor dem Kontakt mit dem Tabak mit einer festen, 2- bis 3- wertigen organischen Säure mit insgesamt 2 bis 6 C-Atomen und/oder deren Monoalkali- oder Monoammoniumsalzen versetzt, die Mischung zur Extraktion der Tabakalkaloide in innigen Kontakt mit dem Tabak bringt und nachfolgend die Tabakalkaloide aus der Mischung abtrennt.

13. Verfahren nach Anspruch 12, dadurch **gekennzeichnet,** daß das Kohlendioxid im Kreislauf geführt wird.

14. Verfahren nach einem der Ansprüche 12 oder 13, dadurch **gekennzeichnet,** daß das im Kreislauf geführte Kohlendioxid vor dem erneuten Kontakt mit dem Tabak mit der organischen Säure und/oder ihren Salzen versetzt wird.